Europäisches Patentamt

European Patent Office

Office européen des brevets

(19)

(11) **EP 1 135 169 B1**

(12) **FASCICULE DE BREVET EUROPEEN**

(45) Date de publication et mention
de la délivrance du brevet:
**12.02.2003 Bulletin 2003/07**

(21) Numéro de dépôt: **99972932.0**

(22) Date de dépôt: **26.11.1999**

(51) Int Cl.7: **A61K 47/48**

(86) Numéro de dépôt international:
**PCT/FR99/02939**

(87) Numéro de publication internationale:
**WO 00/032237 (08.06.2000 Gazette 2000/23)**

(54) **UTILISATION D'UNE COMPOSITION PHARMACEUTIQUE COMPRENANT UN AGENT ANTI-CANCEREUX ET AU MOINS UN PEPTIDE**

VERWENDUNG VON EINER PHARMAZEUTISCHEN ZUSAMMENSETZUNG ENTHALTENDE EIN ANTIKREBS MITTEL UND MINDESTENS EIN PEPTID

USE OF A PHARMACEUTICAL COMPOSITION COMPRISING AN ANTI-CANCER AGENT AND AT LEAST A PEPTIDE

(84) Etats contractants désignés:
**AT BE CH CY DE DK ES FI FR GB GR IE IT LI LU MC NL PT SE**

(30) Priorité: **30.11.1998 FR 9815073**

(43) Date de publication de la demande:
**26.09.2001 Bulletin 2001/39**

(73) Titulaire: **Synt:em (S.A.)**
**30000 Nîmes (FR)**

(72) Inventeurs:
• **TEMSAMANI, Jamal, Résidence de l'Empereur**
**F-30900 Nîmes (FR)**
• **KACZOREK, Michel**
**F-34980 Montferrier sur Lez (FR)**
• **COLIN DE VERDIERE, Annik**
**F-30000 Nîmes (FR)**

(74) Mandataire: **Breese, Pierre et al**
**Breese - Majerowicz - Simonnot**
**3, avenue de l'Opéra**
**75001 Paris (FR)**

(56) Documents cités:
**WO-A-97/12912**     **WO-A-97/19954**
**WO-A-98/46250**     **WO-A-99/07728**

• **DEROSSI D. ET AL: "The third helix of the Antennapedia homeodomain translocates through biological membranes." JOURNAL OF BIOLOGICAL CHEMISTRY., vol. 269, no. 14, 1994, pages 10444-10450, XP002114618 AMERICAN SOCIETY OF BIOLOGICAL CHEMISTS, BALTIMORE, MD., US ISSN: 0021-9258 cité dans la demande**

**Description**

La présente invention concerne l'utilisation de peptides pour la vectorisation d'agents anticancéreux pour des applications dans le traitement et/ou la prévention des cancers et plus particulièrement dans le domaine de la chimiorésistance.

Un problème important auquel se heurte la pharmacologie des produits anticancéreux est la résistance intrinsèque ou acquise des cellules cancéreuses à ces produits. En effet, on observe que les cellules cancéreuses de nombreux patients atteints de cancer deviennent ou sont résistantes aux agents anticancéreux, ce qui se traduit par l'apparition de nouvelles évolutions tumorales chez le patient. Les épithéliomes digestifs, les mélanomes, les cancers du rein sont des exemples de chimiorésistance innée. Les leucémies, les cancers du sein, les cancers des bronches à petites cellules chez l'adulte, les neuroblastomes chez l'enfant, répondent généralement bien en début de traitement mais, dans une proportion importante, deviennent progressivement résistants aux traitements.

La résistance primaire peut-être liée à des mécanismes d'inhibition du transport transmembranaire, d'inhibition de l'activation des prodrogues, à la modification de l'enzyme cible, de voies métaboliques, à des phénomènes de réparation et d'inactivation.

Les phénomènes de résistance acquises sont multiples. L'une de ces résistances est la résistance multidrogue (multidrug resistance ou MDR). La MDR est associée à une diminution de la rétention intracellulaire des médicaments de mode d'action cependant différents (Chen et al., 1986, Cell 47, 381-389 ; Krishan et al., 1997, Cytometry 29, 279-285 ; Riordan et al., 1985, Nature 316, 817-819).

Une protéine associée à la membrane, la P-glycoprotéine ou "P-gp", est l'expression phénotypique du gène MDR. Cette protéine agit comme une pompe énergie dépendante transportant des drogues cytotoxiques vers l'extérieur de la cellule avant qu'elles ne produisent leurs effets. L'expression de cette protéine conduit la cellule tumorale à résister à des concentrations élevées d'agents cytotoxiques tels que la doxorubicine, la daunomycine, l'actinomycine D, la vinblastine, la vincristine, la mitomycine C, l'etoposide, la téniposide, etc.... La protéine P-gp est exprimée dans les cellules normales comme dans celles du tractus gastro-intestinal, du foie ou du rein, où l'on pense qu'elle sert à éliminer les toxines ou des médicaments. Elle serait aussi responsable de la faible pénétration dans le cerveau de nombreux médicaments.

Une des priorités de la recherche dans le domaine du cancer, est donc de trouver des moyens efficaces pour circonvenir à l'expression et l'efficacité du phénotype de la résistance multidrogue, afin de limiter les échecs des chimiothérapies. Plusieurs études ont été réalisés pour trouver des agents qui inhibent la résistance liée à la P-gp et à restaurer totalement ou partiellement, l'activité antitumorale du produit cytotoxique. De tels agents sont appelés chimiosensibilisateurs ou modulateurs de la P-gp. Ces agents agissent soit directement, en interférant, par compétition ou encombrement stérique, au niveau des sites de fixation des agents cytotoxiques ou indirectement en inhibant la protéine responsable par divers mécanismes. Toute une série de produits sont capables d'inhiber la résistance multidrogues, tels que des inhibiteurs des canaux calciques, phénothiazines, quinidine, agents anti-paludéens, anti-oestrogène, cyclosporine. Cependant la toxicité de ces produits limite pour l'instant leur utilisation clinique.

Afin de palier cet inconvénient, il a été proposé dans l'art antérieur des systèmes de transport de certains agents anticancéreux, notamment la doxorubicine, utilisant la transferrine (Barabas K. et al., 1992, The Journal of Biological Chemistry, 267(13) : 9347-9442), le dextran (Ueda Y. et al., 1989, Chem. Pharm. Bull., 37(6) : 1639-41 ; Sheldon K. et al., 1989, Anticancer Research, 9(3) : 637-642), des anticorps (Hurwitz E. et al., 1975, Cancer Research, 35 : 1175-1181), des microspheres (Rogers K. E. et al., 1983, Cancer Research, 43 : 2741-2748 ; Jeanneson P. et al., 1990, Cancer Research, 50, 1231-1236), des polymères (Tokoyama M. et al., 1990, Cancer Research, 50 : 1693-1700), ou des fragments de protéines (Ohkawa K. et al., 1993, British Journal of Cancer, 67 : 247-8 ; Asakura T. et al., 1997, Anticancer Drug, 8(2) : 199-203). Il a aussi été proposé des moyens de transport des agents anti-cancéreux par séquestration dans des liposomes ou des nanoparticules (Kruh G. D. et Goldstein L. J., 1993, Curr. Opin. Oncol., 5(6) : 1029-34 ; Cuvier C. et al., 1992, Biochemical Pharmacology, 44 : 509-517). Mais ces systèmes ne se sont pas avérés performants du fait notamment de leur toxicité, d'une faible spécificité vis-à-vis des cellules cancéreuses, de la mauvaise stabilité du produit fini au caurs de la conservation, et d'une faisabilité difficile.

La présente invention vise donc à offrir un nouveau moyen efficace et non toxique pour lutter contre le problème de résistance multidrogue. Ce but est atteint grâce à l'utilisation de peptides pour la vectorisation d'agents cytotoxiques jusqu'à la cible cancéreuse, lesdits peptides permettant en outre de faire échapper lesdits agents aux différents mécanismes de résistance et particulièrement à la pompe P-gp.

Il a été décrit dans l'art antérieur de nombreux peptides capables de traverser les membranes des eucaryotes de manière très rapide et tels que les peptides suivants : Protégrine, Antennapedia, Tachyplésine, Transportan, etc....

Parmi ceux-ci, certains présentent des propriétés cytolytiques. Ces peptides dénommés peptides antibiotiques sont notamment les Protégrines et Tachyplésines. Les Protégrines et Tachyplésine sont des peptides antibiotiques naturels dont la structure est de type épingle à cheveux maintenue par des ponts disulfures. Ces ponts jouent un rôle important dans l'activité cytolytique observée sur cellules humaines.

Selon leur structure, les peptides antibiotiques peuvent être classés en trois grandes familles :

- Les peptides antibiotiques à hélices alpha amphipatiques : cécropines et maganines (Maloy, W. L. et al., 1995, BioPolymer 37, 105-122).
- Les peptides antibiotiques à feuillets bêta réunis par des ponts disulfures : défensines (Lehrer, R. I. et al., 1991, Cell 64:229-230 ; Lehrer, R. I. et al., 1993, Ann. Rev. Immunol. 11:105-128), protégrines (Kokryakov, V. N. et al., 1993, FEBS 337:231-236), tachyplésines (Nakamura, T. et al., 1988, J. Biol. Chem. 263:16709-16713 ; Miyata, T et al., 1989, J. Biochem. 106:663-668).
- les peptides antibiotiques à chaînes déstructurées contenant de nombreux coudes liés à la présence de multiples prolines : bacténécines et PR39 (Frank, R. W. et al., 1991, Eur. J. Biochem. 202, 849-854).

On désigne sous le nom de protégrines un ensemble de cinq peptides désignés PG-1, PG-2, PG-3, PG-4 et PG-5 dont les séquences sont données ci-dessous, étroitement apparentés et isolés de leucocytes de porc (V.N. Kokryakov & col. FEBS lett. 327, 231-236) :

$$
\begin{aligned}
&\text{PG-1 : RGGRLCYCRRRFCVCVGR-NH}_2 \\
&\text{PG-2 : RGGRLCYCRRRFCICV..-NH}_2 \\
&\text{PG-3 : RGGGLCYCRRRFCVCVGR-NH}_2 \\
&\text{PG-4 : RGGRLCYCRGWICFCVGR-NH}_2 \\
&\text{PG-5 : RGGRLCYCRPRFCVCVGR-NH}_2
\end{aligned}
$$

Les tachyplésines (Tamura, H. et al., 1993, Chem. Pharm. Bul. Tokyo 41, 978-980), désignées T1, T2 et T3 et les polyphémusines (Muta, T., 1994, CIBA Found. Sym. 186, 160-174), désignées P1 et P2, dont les séquences sont données ci-dessous, sont des peptides homologues isolés de l'hémolymphe de deux crabes, *Tachyplesus tridentatus* pour les tachyplésines T1, T2 et T3 et *Limmulus polyphemus* pour les polyphémusines P1 et P2 :

$$
\begin{aligned}
&\text{P1 : RRWCFRVCYRGFCYRKCR-NH}_2 \\
&\text{P2 : RRWCFRVCYKGFCYRKCR-NH}_2
\end{aligned}
$$

Protégrines, tachyplésines et polyphémusines contiennent une forte proportion de résidus basiques (lysines et arginines) et possèdent quatre cystéines qui forment deux ponts disulfures paralléles. Ces trois familles de peptides présentent également des homologies avec certaines défensines et en particulier avec la défensine humaine NP-1 (Kokryakov, V. N. et al., 1993, Febs Let. 327, 231-236).

Ainsi, dans le cadre de ces travaux de recherche, la Demanderesse a découvert que la réduction irréversible de ces ponts disulfures permet d'obtenir des peptides linéaires, ayant la capacité de traverser rapidement les membranes des cellules de mammifères par un mécanisme passif ne faisant pas appel à un récepteur membranaire. Ces peptides linéaires sont non-toxiques, et sans activité lytique, et en conséquence, ils constituent un nouveau système de vectorisation de substances actives dans les domaines thérapeutique ou diagnostic. Les travaux et résultats concernant ces peptides linéaires et leur utilisation comme vecteurs de substances actives sont décrits dans la demande de brevet français de la Demanderesse déposée le 12 Août 1998 sous le No. 97/10297.

Les peptides issus de la famille Antennapedia sont des dérivés du facteur de transcription de l'homéodomaine Antennapedia de la mouche drosophile et sont par exemple décrits dans les demandes de brevet internationales PCT publiées sous les No. WO91/18981 et WO97/12912. La séquence de ces peptides présente la particularité d'être hautement conservée dans toutes les homéoprotéines. Ces peptides sont composés de trois hélices alpha et sont capables de se transloquer au travers de la membrane cellulaire. Le plus petit fragment de l'homeodomaine capable de traverser les membranes est un peptide de 16 acides aminés (Prochiantz, 1996, Curr. Opin. In Neurob. 6, 629-634 ; Derossi et al., 1994, J. Biol. Chem. 269, 10444-10450).

Les travaux de recherche réalisés dans le cadre de la présente invention ont maintenant permis à la demanderesse de montrer que ces peptides linéaires, c'est à dire dépourvus de pont disulfure, peuvent être utilisés comme système de vectorisation très efficace permettant de d'amener une substance anti-cancéreuse jusqu'à une cible et de faire traverser à ladite substance la membrane cellulaire de façon à conduire celle-ci jusque dans un compartiment cellulaire tel que le cytoplasme ou le noyau. En outre, de façon surprenante, la Demanderesse a découvert, qu'en plus de leur capacité de vectorisation, certains des ces peptides peuvent être utilisés pour empêcher l'expression de résistance intrinsèque ou acquise des cellules cancéreuses vis-à-vis de ces agents, ci-après aussi désigné chimiorésistance, notamment de contrer le phénomène de résistance multidrogue (MDR) et de permettre à ces agents d'échapper à la pompe P-gp.

L'invention a donc plus particulièrement pour objet une composition pharmaceutique destinée au traitement et/ou à la prévention des cancers comprenant au moins un agent anti-cancéreux, caractérisée en ce que ledit agent anti-cancéreux est associé dans la composition avec au moins peptide linéaire capable de transporter ledit agent dans les cellules cancéreuses et d'empêcher l'apparition d'une chimiorésistance vis-à-vis dudit agent, ledit peptide répondant à l'une des formules (I), (II) ou (III) suivantes:

$$X_1\text{-}X_2\text{-}X_3\text{-}X_4\text{-}X_5\text{-}X_6\text{-}X_7\text{-}X_8\text{-}X_9\text{-}X_{10}\text{-}X_{11}\text{-}X_{12}\text{-}X_{13}\text{-}X_{14}\text{-}X_{15}\text{-}X_{16} \qquad \text{(I)}$$

dans laquelle formule (I), les résidus $X_1$ à $X_{16}$ sont des résidus d'acides aminés dont 6 à 10 d'entre-eux sont des acides aminés hydrophobes et $X_6$ est le tryptophane,

$$BXXBXXXXBBBXXXXXXB \qquad \text{(II)}$$

$$BXXXBXXXBXXXXBBXB \qquad \text{(III)},$$

dans lesquelles formules (II) et (III) :

-    les groupes B, identiques ou différents, représentent un résidus d'acide aminé dont la chaîne latérale porte un groupement basique, et
-    les groupes X, identiques ou différents, représentent un résidus d'acide aminé aliphatique ou aromatique,

ou lesdits peptides de formules (I), (II), (III) sous forme rétro, constitués d'acides aminés de configuration D et/ou L, ou un fragment de ceux-ci constitué d'une séquence d'au moins 5 et de préférence d'au moins 7 acides aminés successifs des peptides de formules (I), (II) ou (III), dès lors bien entendu que ce fragment présente les propriétés de vectorisation sans toxicité pour les cellules.

Les peptides de formule (I) dérivent de la famille Antennapedia. Dans les peptides de formules (I), les acides aminés hydrophobes sont l'alanine, la valine, la leucine, l'isoleucine, la proline, la phénylalanine, le tryptophane, la tyrosine et la méthionine, et les autres acides aminés sont des acides aminés :

-    non-hydrophobes qui peuvent être des acides aminés non polaires comme la glycine, ou polaires comme la sérine, la thréonine, la cystéine, l'asparagine, la glutamine, ou
-    acides (acide aspartique ou glutamique), ou
-    basiques (lysine, arginine ou histidine), ou
-    une association d'acides aminés de ces trois catégories.

Parmi les peptides de formule (I), on préfère ceux comprenant 6 acides aminés hydrophobes et 10 acides aminés non-hydrophobes.

Les peptides linéaires de formule (II) dérivent de la famille Protégrine et les peptides linéaires de formule (III) dérivent de la famille Tachyplésine. Parmi les peptides de formules (II) et (III), on préfère ceux dans lesquels :

-    B est choisi parmi l'arginine, la lysine, l'acide diaminoacétique, l'acide diaminobutyrique, l'acide diaminopropionique, l'ornithine, et
-    X est choisi parmi la glycine, l'alanine, la valine, la norleucine, l'isoleucine, la leucine, la cystéine, la cystéine[Acm], la penicillamine, la méthionine, le serine, la thréonine, l'asparagine, la glutamine, la phényalanine, l'histidine, le tryptophane, la tyrosine, la proline, l'Abu, l'acide amino-1-cyclohexane carboxylique, l'Aib, la 2-aminotétraline carboxylique, la 4-bromophényalanine, tert-Leucine, la 4-chlorophénylalanine, la bêta-cyclohexyalanine, la 3,4-dichlorophényalanine, la 4-fluorophényalanine, l'homoleucine, la bêta-homoleucine, l'homophényalanine, la 4-méthylphényalanine, la 1-naphtyalanine, la 2- naphtyalanine, la 4-nitrophényalanine, la 3-nitrotyrosine, la norvaline, la phénylglycine, la 3-pyridyalanine, la [2-thiényl]alanine.

Dans les peptides de formules (I), (II) ou (III), B, X et $X_1$ à $X_{16}$ peuvent être des acides aminés naturels ou non, y compris des acides aminés de configuration D.

Des peptides préférés utilisés selon l'invention sont choisis parmi ceux dont les séquences en acides aminés sont les suivantes :

RGGRLSYSRRRFSTSTGR

RGGRLSYSRRRFSVSVGR

KWSFRVSYRGISYRRSR

RRLSYSRRRF

Rqikiwfqnrrmkwkk

CENIKIWLSLRSYLKRR

RGGRLAYLRRRWAVLVGR

où les lettres minuscules représentent des acides aminés sous forme D.

L'association d'un agent anti-cancéreux et d'un peptide défini ci-dessus dans les compositions de l'invention consiste avantageusement en un couplage qui peut être réalisé par tout moyen de liaison acceptable compte tenu de la nature chimique, de l'encombrement et du nombre d'agent anti-cancéreux et de peptide associés. Il peut s'agir de liaisons covalentes, hydrophobes ou ioniques, clivables ou non-clivables dans les milieux physiologiques ou à l'intérieur de la cellules.

Le couplage peut être effectué en n'importe quel site du peptide, dans lequel des groupements fonctionnels tels que -OH, -SH, -COOH, $-NH_2$ sont naturellement présents ou ont été introduits. Ainsi, un agent anti-cancéreux peut être lié au peptide au niveau des extrémités N-terminale ou C-terminale ou bien au niveau des chaînes latérales du peptide.

De même, le couplage peut être effectué en n'importe quel site de l'agent actif, où par exemple des groupements fonctionnels tels que -OH, -SH, -COOH, $-NH_2$ sont naturellement présents ou ont été introduits.

Ainsi, l'invention se rapporte à l'utilisation de composés répondant à la formule (IV) suivante :

$$A\ (-)_m\ (B)_n \hspace{4cm} (IV)$$

dans laquelle

- A représente un peptide tel que défini précédemment,
- B représente un agent anti-cancéreux,
- n est 1 ou plus, et de préférence jusqu'à 10 avantageusement jusqu'à 5,
- $(-)_m$ représente la liaison, ou linker, entre A et B, où m est 1 ou plus, et de préférence jusqu'à 10 avantageusement jusqu'à 5,

pour la préparation d'un médicament destiné au traitement et/ou à la prévention des cancers sans induire de chimiorésistance.

Les composés de formule (IV) peuvent être préparés par synthèse chimique ou en utilisant des techniques de biologie moléculaire.

On peut utiliser pour les synthèses chimiques des appareils commerciaux permettant d'incorporer des acides aminés non-naturels, tels que les énantiomères D et des résidus ayant des chaînes latérales ayant des hydrophobicités et des encombrements différents de ceux de leurs homologues naturels. Au cours de la synthèse, il est évidemment possible de réaliser un large éventail de modifications, par exemple introduire sur le N-terminal un lipide, par exemple prenyl ou myristyl, de façon à pouvoir ancrer le peptide de l'invention et donc le composé de formule (IV) à une membrane lipidique telle que celle d'un liposome constitué de lipides. Il est également possible de remplacer une ou plusieurs liaisons peptidiques (-CO-NH-) par des structures équivalentes comme $-CO-N(CH_3)-$, $-CH_2-CH_2-$, $-CO-CH_2-$, ou bien d'intercaller des groupes comme $-CH_2-$, -NH-, -O-.

On peut également obtenir les composés de formule (IV) ou partie de ceux-ci de nature protéique à partir d'une séquence d'acide nucléique codant pour celui-ci. La présente invention a aussi pour objet une molécule d'acide nucléique comprenant ou constituée par une séquence nucléique codant pour un peptide linéaire dérivé de peptide antibiotique. Plus particulièrement l'invention concerne une molécule d'acide nucléique comprenant au moins une séquence codant pour un composé de formule (IV) ou une partie de celui de nature protéique. Ces séquences d'acides nucléiques peuvent être des ADN ou ARN et être associées à des séquences de contrôle et/ou être insérées dans des vecteurs. Le vecteur utilisé est choisi en fonction de l'hôte dans lequel il sera transféré; il peut s'agir de tout vecteur comme un plasmide. Ces acides nucléiques et vecteurs sont utiles pour produire les peptides et les composés de formule (IV) ou partie de ceux-ci de nature protéique dans un hôte cellulaire. La préparation de ces vecteurs ainsi que la production ou l'expression dans un hôte des peptides ou des composés de formule (IV) peuvent être réalisées par les techniques de biologie moléculaire et de génie génétique bien connues de l'homme du métier.

Comme indiqué précédemment, les travaux de recherche ont mis en évidence que de manière surprenante, les peptides définis ci-dessus, sont capables non seulement de transporter l'agent anticancéreux jusque dans les cellules cancéreuses, mais aussi d'empêcher l'apparition de chimiorésistance vis-à-vis de cet agent. En conséquence, l'invention a également pour objet un procédé d'inhibition de la capacité éventuelle d'un agent anticancéreux à induire une chimiorésistance chez un sujet ayant reçu ledit agent consistant à associer ledit agent à au moins un peptide de formules (I), (II) ou (III) par tout moyen approprié comme notamment ceux décrits précédemment.

L'invention a donc également pour objet l'utilisation d'un composé de formule (IV) tel que défini précédemment pour la préparation d'un médicament anticancéreux capable en outre de prévenir l'apparitio d'une chimiorésistance.

Selon une forme préférée de mise en oeuvre de l'utilisation ci-dessus, ledit peptide est associé dans le médicament avec l'agent anti-cancéreux par une liaison du type de celle décrite précédemment. Tout préférentiellement cette liaison est clivable sélectivement dans le milieu cellulaire. Ledit médicament comprend un véhicule pharmaceutiquement acceptable et compatible avec le mode d'administration adopté.

Les agents anti-cancéreux entrant dans le cadre de la présente invention sont tous ceux utilisés ou utilisables en chimiothérapie, comme par exemple de manière non limitative : la doxorubicine, la daunomycine, l'actinomycine D, la vinblastine, la vincristine, la mitomycine C, L'etoposide, le téniposide, le taxol, le taxotère, le méthotrexate, etc.... Bien entendu, l'invention concerne plus particulièrement les agents anti-cancéreux dont ont déjà mis en évidence l'apparition d'une chimiorésistance chez les individus qui y ont été exposés.

On entend aussi par agent anti-cancéreux, dans le cadre de la présente invention, des substances actives contre la P-gp ou le gène codant celle-ci, et plus particulièrement des anticorps ou partie d'anticorps, des acides nucléiques et oligonucléotides ou des ribozymes. En effet, la présente invention concerne également l'association d'oligonucléotide antisens avec les peptides précédemment décrits pour bloquer l'expression de la P-gp et donc utile dans le traitement ou la prévention des cancers en s'opposant au phénomène de résistance multidrogue.

Les compositions de l'invention contenant des composés de formule (IV) et avantageusement un véhicule pharmaceutiquement acceptable peuvent être administrées par différentes voies comme par exemple de manière non limitative, les voies intraveineuse, intramusculaire, sous cutanée, etc....

D'autres avantages et caractéristiques de l'invention apparaîtront dans les exemples qui suivent concernant la préparation de composés de formule (IV) où l'agent anticancéreux est la doxorubicine et l'effet de la vectorisation de la doxorubicine sur son internalisation.

I - CONDITIONS EXPÉRIMENTALES.

1) Synthèse Chimique.

Plusieurs peptides ont été synthétisés et leur internalisation a été testée dans plusieurs lignées cellulaires. De façon générale, les propriétés physico-chimiques des peptides ont été modifiées, et les résultats obtenus montrent que suivant la modification, certains peptides pénètrent beaucoup mieux que d'autres, comme les peptides des composés No. 1 à 6 du tableau I ci-après. Il a également été observé que certains peptides pénètrent plus rapidement dans un type cellulaire que dans d'autres, ce qui indique un tropisme cellulaire.

a) Préparation de Doxorubicine-Succ-Peptides.

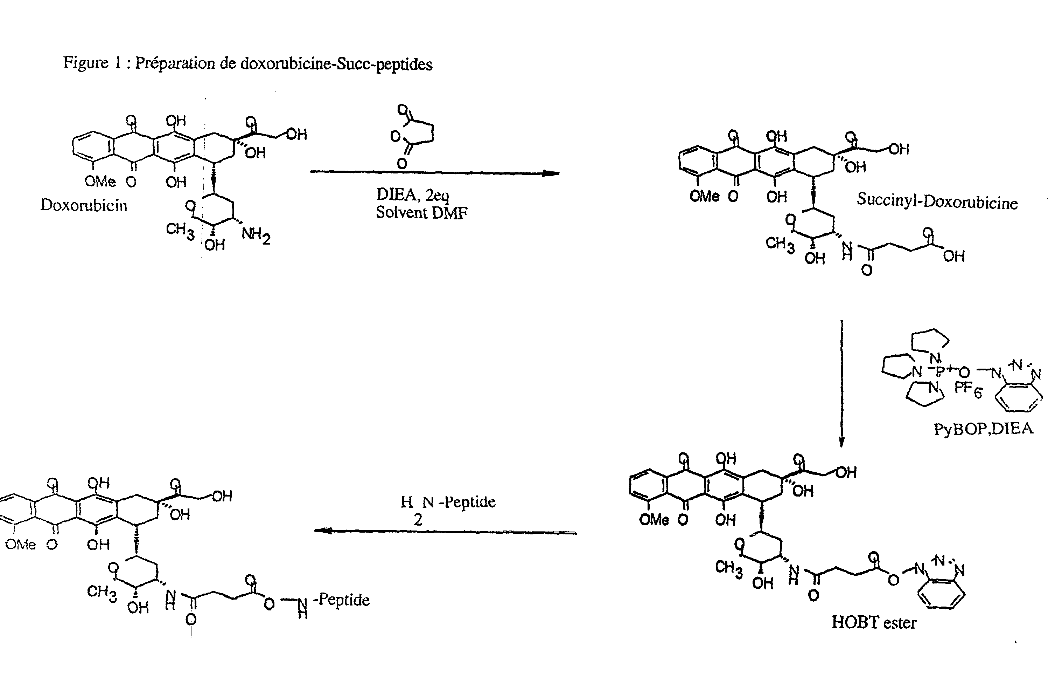

Comme indiqué dans le schéma de synthèse de la figure 1, le couplage de doxorubicine sur un peptide par l'intermédiaire du maillon succinique est effectué en 3 étapes :

Au chlorhydrate de doxorubicine (1 eq), solubilisé dans diméthylformaminde (DMF) en présence de Diisopropyléthylamine (DIEA, 2 eq),.est ajouté l'anhydride succinique (1,1eq, dissous dans DMF).

Après une incubation de 20 min à température ambiante, l'hémisuccinate de doxorubicine ainsi formé est ensuite activé par addition de PyBOP (Benzotriazol-1-yl-oxopyrrolidinephosphonium Hexafluorophosphate 1,1eq dans DMF) et DIEA (2 eq). Ce second mélange réactionnel est incubé 20 min.

Le peptide (1.2 eq dans DMF) est ensuite ajouté au mélange réactionnel, et se couple spontanément sur l'hémisuccinate de doxorubicine activé au cours d'une incubation supplémentaire de 20 min.

Le produit de couplage est ensuite purifié sur HPLC (Chromatographie liquide haute pression) préparative, puis lyophilisé.

Chacune des étapes, ainsi que le produit final sont contrôlés par HPLC analytique et spectrométrie de masse.

b) Préparation de Doxorubicine-SMP-3MP-Peptide.

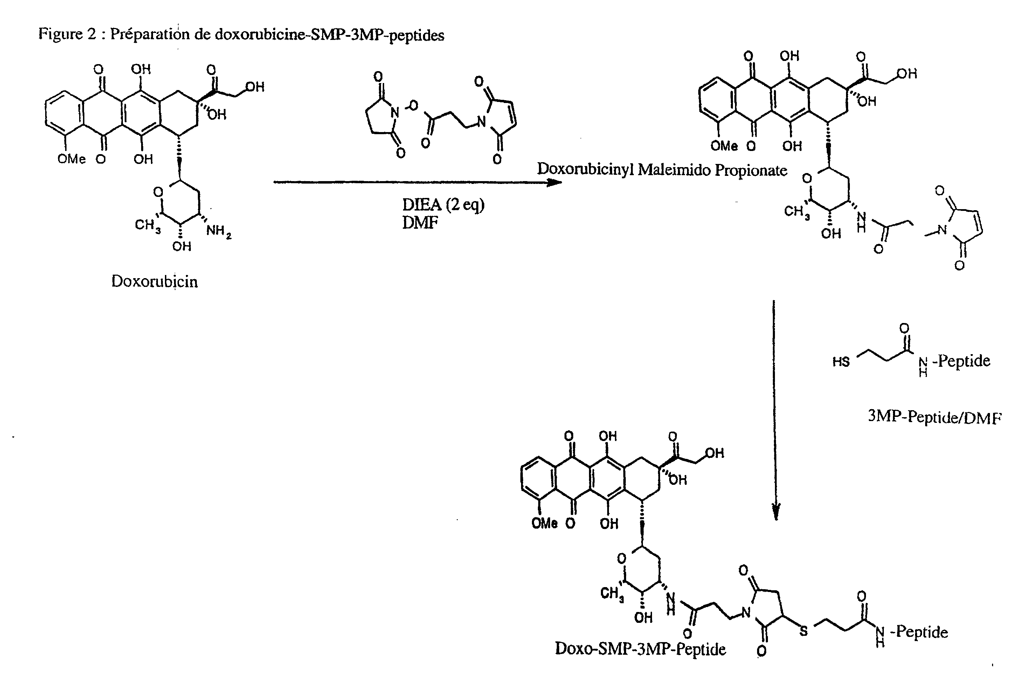

Comme indiqué dans le schéma de synthèse de la figure 2, le couplage De la doxorubicine sur un peptide porteur de fonction thiol est effectué en 2 étapes :

Au chlorhydrate de doxorubicine (1 eq), solubilisé dans diméthylformaminde (DMF) en présence de Diisopropyléthylamine (DIEA, 2 eq), est ajouté le N-hydroxy-Succinimidyl-Maleimido-Propionate (SMP, 1 eq dans dissous dans DMF).

Le peptide porteur d'une fonction thiol (1.2 eq dans DMF) est ensuite ajouté au mélange réactionnel, et se couple spontanément sur le maléimidopropionate de doxorubicine au cours d'une incubation supplémentaire de 20 min.

Le produit de couplage est ensuite purifié sur HPLC (Chromatographie liquide haute pression) préparative, puis lyophilisé.

Chacune des étapes, ainsi que le produit final sont contrôlés par HPLC analytique et spectrométrie de masse.

2) Les produits testés.

Les produits testés sont rapportés dans le tableau I ci-dessous.

## Tableau I

| Composé | |
|---|---|
| 1 | doxo-CO-(CH2)$_2$-CO- RGGRLSYSRRRFSTSTGR |
| 2 | doxo-CO-(CH2)$_2$-CO- RGGRLSYSRRRFSVSVGR |
| 3 | doxo-CO-(CH2)$_2$-CO- KWSFRVSYRGISYRRSR |
| 4 | doxo-CO-(CH2)$_2$-CO- RRLSYSRRRF |
| 5 | doxo-SMP-3MP-rqikiwfqnrrmkwkk |
| 6 | doxo-SMP-CENIKIWLSLRSYLKRR |
| 7 | doxo-CO-(CH2)2-CO-RGGRLAYLRRRWAVLVGR |

doxo = doxorubicine

CO-(CH2)$_2$-CO = Linker succinate

SMP-3MP    =    Linker    SuccinimydylMaleimido-Propionate-3-MercaptoPropionate.

3) Culture Cellulaire.

Les cellules de leucémie myéloïde chronique K562 sensibles et les cellules résistantes K562/ADR ainsi que les cellules HL60/R10 de leucémie promyéloïde sont d'origine humaine et ont été obtenues commercialement auprès de l'ATCC. Les cellules sont ensemencées à environ $10^4$ cellules par puits, 24 h avant l'addition des produits. Les cellules sont maintenues en culture à 37°C dans une atmosphère à 95% d'humidité et 5% de $CO_2$ dans un milieu OptiMem.

4) Internalisation.

Les cellules K562 sensibles et les cellules résistantes K562/ADR sont incubées soit avec la doxorubicine libre soit avec la doxorubicine vectorisée à une concentration de 3 μM. Après 30 minutes d'incubation à 37°C, les cellules sont lavées trois fois au PBS. L'internalisation des produits est ensuite analysée par cytométrie en flux. Les échantillons sont analysés par un cytomètre de flux équipé d'un laser argon de 15 mA. La fluorescence émise est enregistrée sur une échelle logarithmique à (575 NM) nm après une excitation à (488) nm. La fluorescence est mesurée sur 10000 cellules sélectionnées suivant les paramètres de taille (SS) et de granulosité (FS). Les résultats sont exprimés en pourcentage de cellules positives du logarithme de l'intensité de fluorescence. Les résultats sont analysés par le logiciel Cell Quest.

5) Cytotoxicité.

Les cellules K562 sensibles et les cellules résistantes K562/ADR sont incubées soit avec la doxorubicine libre soit avec la doxorubicine vectorisée à des concentrations croissantes. Après 48 heures en culture en présence des produits. A la fin du temps de culture, le MTT (3-(4,5-diméthylthiazole-2-yl)-2,5-diphényltétrazolium bromure) est rajouté dans les puits et les plaques de culture sont ensuite incubées pendant 4 heures dans l'étuve. Le dépôt cristallin de formazan résultant est alors dissout par addition de 200 μl de DMF/SDS. La densité optique (DO) est mesurée à 550 nm (référence 630 nm) en utilisant un lecteur de microplaques.

La représentation graphique des pourcentages de DO des puits traités en fonction de la concentration de produits permet de déterminer la $IC_{50}$. Celle-ci correspond à la concentration du produit inhibant 50% de la croissance.

Les valeurs de la $IC_{50}$ permettent de quantifier le facteur de résistance (F. Rés) des lignées résistantes par le rapport suivant :

$$\text{F.Rés} = \frac{IC_{50} \text{ du cytotoxique de la lignée résistante}}{IC_{50} \text{ du cytotoxique de la lignée parentale sensible}}$$

Le facteur de réversion (F.Rev) correspond à l'effet d'un modulateur (le vecteur) sur la sensibilité des cellules aux agents antitumoraux selon le rapport suivant :

$$\text{F.Rés} = \frac{IC_{50} \text{ du cytotoxique seul}}{IC_{50} \text{ du cytotoxique vectorisé}}$$

II - RESULTATS.

1) Internalisation.

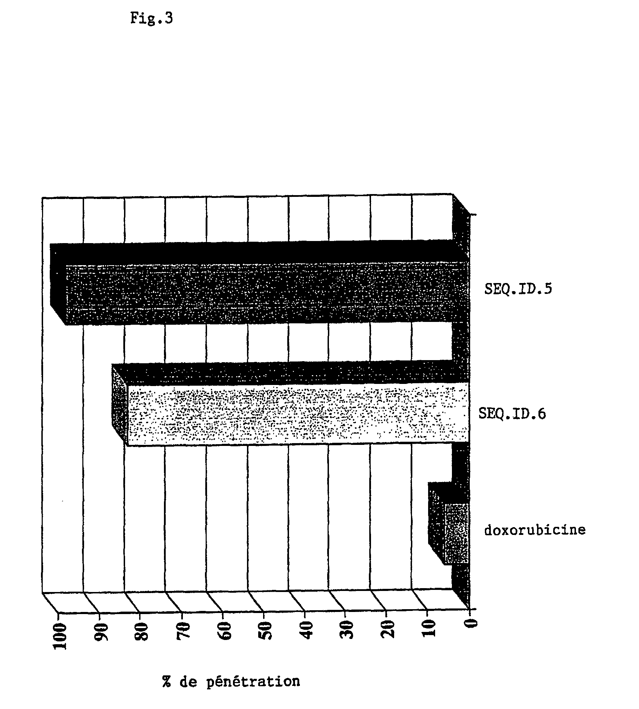

Les cellules sensibles K562 et résistantes K562/ADR sont incubées soit avec la doxorubicine libre soit avec la doxorubicine vectorisée. Après 30 min d'incubation, l'internalisation des produits est mesurée par cytométrie de flux. La Figure 3 en annexe montre que dans les cellules résistantes K562/ADR, seulement 5,86% des cellules sont positive alors que quand elle est vectorisée (par exemple sous la forme du composé No. 5 du tableau I) 98% des cellules sont positives, ce qui indique une nette amélioration de pénétration.

2) Cytotoxicité.

La sensibilité des cellules aux agents antitumoraux a été mesuré par le test MTT dans les conditions expérimentales définies ci dessus pour lesquelles la relation entre la densité optique et le nombre de cellules viables est linéaire.

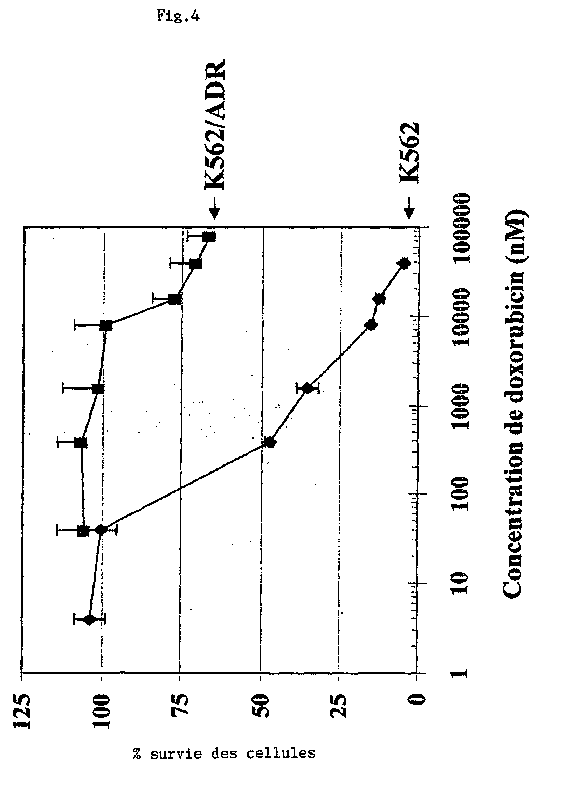

L'activité de la doxorubicine dans les cellules sensibles (K562) et dans les cellules résistantes (K562/ADR) a tout d'abord été observée. Les cellules sont incubées avec des concentrations croissantes de doxorubicine et après 24 heures d'incubation, la survie des cellules est mesurée par le test MTT. Comme le montre la Figure 4, les cellules K562/ADR sont bien résistantes à la doxorubicine. Par exemple, à une concentration de 8 μM, seulement 15% des cellules sensibles survivent alors que les cellules résistantes survivent à 100%.

Puis, le cytotoxicité de la doxorubicine vectorisée a été analysée. Le tableau II ci-dessous représente les concentrations en produits entraînant 50% d'inhibition de croissance ($IC_{50}$) déterminées sur la lignée sensible (K562) et la lignée résistante (K562/ADR).

Tableau II

| Lignées | K562 | K562/ADR | F.Rés | F.Rev |
|---|---|---|---|---|
| Doxo libre | 0,9 µM | 25 µM | 28 | |
| Composé 1 | 15 µM | 5-10 µM | 0,7 | 2,5 |
| Doxo libre | 0,15 µM | 15,5 µM | 100 | |
| Composé 2 | 3, 6 µM | 3,2 µM | 0,9 | 5 |
| Doxo libre | 0,45 µM | 65 µM | 144 | |
| Composé 3 | 2 µM | 1,5 µM | 0,8 | 43 |
| Doxo libre | ND | 55 µM | | |
| Composé 4 | 20 µM | 20 µM | 1 | 2,8 |
| Doxo libre | 0,4 µM | 70 µM | 175 | |
| Composé 5 | 1,5 µM | 2 µM | 1,3 | 35 |
| Doxo libre | 0,3 µM | 70 µM | 233 | |
| Composé 6 | 5 µM | 5,5 µM | 1,1 | 12,8 |
| Doxo libre | 0,1 µM | > 78 uM | > 780 | |
| Composé 7 | 3,5 µM | 3 µM | 0,85 | > 26 |

Ces résultats montrent que les cellules K562/ADR sont bien résistantes à la doxorubicine seule. La vectorisation de la doxorubicine par un peptide-vecteur permet de circonvenir au problème de résistance multidrogue. Par exemple, dans un cas, la $IC_{50}$ de la doxorubicine libre dans les cellules résistantes est de 70 µM alors que quand elle est vectorisée (composé 5) la $IC_{50}$ n'est que de 2 µM.

Ces résultats montrent également que les produits vectorisés ne sont pas excrétés par la pompe P-gp puisque la même $IC_{50}$ est obtenue dans les cellules sensibles et les cellules résistantes et le Facteur de résistance est presque 1.

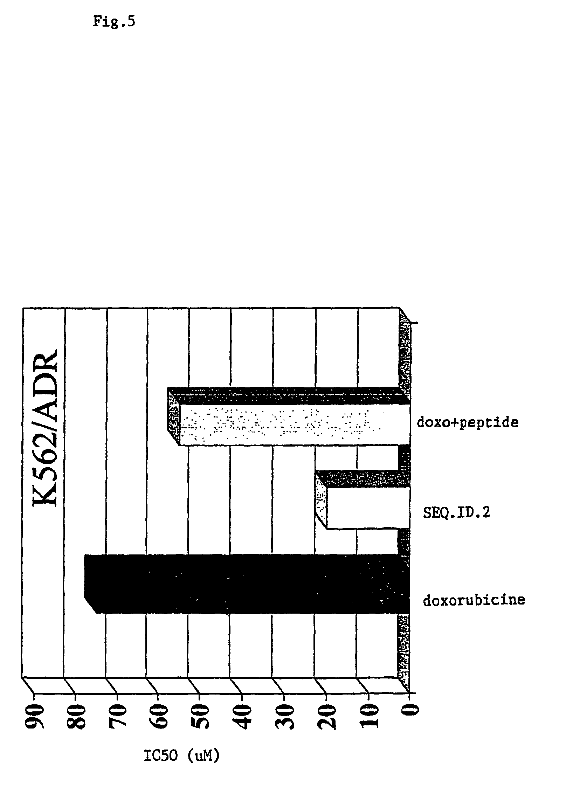

Pour être sûr que la cytotoxicité observée pour la doxorubicine vectorisée ne provient pas du peptide tout seul, une expérience a été réalisée en comparant l'activité de la doxorubicine vectorisée (composé 2) avec la doxorubicine rajoutée au peptide mais non couplée. La figure 5 en annexe montre que l'$IC_{50}$ de la doxorubicine vectorisée (composé 2) est de 19 µM alors que celle de la doxorubicine rajoutée au vecteur est d'environ 50 µM, démontrant ainsi la nécessité de vectorisation pour réduire la résistance des cellules.

Le même type d'expérience a été réalisée dans une autre lignée cellulaire résistante à la doxorubicine (HL60/R10). Les résultats de cytoxicité dans les cellules HL60 avec le composé No. 2 sont rapportés dans le tableau III ci-dessous.

Tableau III

| | HL60/R10 | F. Rev |
|---|---|---|
| Doxo libre | 40 µm | |
| Composé No. 2 | 25 µm | 1,6 |

**Revendications**

1.    Utilisation d'un peptide répondant à l'une des formules (I), (II) ou (III) suivantes:

$$X_1\text{-}X_2\text{-}X_3\text{-}X_4\text{-}X_5\text{-}X_6\text{-}X_7\text{-}X_8\text{-}X_9\text{-}X_{10}\text{-}X_{11}\text{-}X_{12}\text{-}X_{13}\text{-}X_{14}\text{-}X_{15}\text{-}X_{16} \qquad (I)$$

dans laquelle formule (I), les résidus $X_1$ à $X_{16}$ sont des résidus d'acides aminés dont 6 à 10 d'entre-eux sont des acides aminés hydrophobes et $X_6$ est le tryptophane,

$$BXXBXXXXBBBXXXXXXB \qquad (II)$$

$$BXXXBXXXBXXXXBBXB \qquad (III),$$

dans lesquelles formules (II) et (III) :

- les groupes B, identiques ou différents, représentent un résidus d'acide aminé dont la chaîne latérale porte un groupement basique, et
- les groupes X, identiques ou différents, représentent un résidus d'acide aminé aliphatique ou aromatique,

ou lesdits peptides de formules (I), (II), (III) sous forme rétro, constitués d'acides aminés de configuration D et/ou L, ou un fragment de ceux-ci constitué d'une séquence d'au moins 5 et de préférence d'au moins 7 acides aminés successifs des peptides de formules (I), (II) ou (III),

pour la préparation d'un médicament destiné au traitement et/ou la prévention de cancers chimiorésistants, ledit peptide étant associé dans le médicament avec au moins un agent anticancéreux pour vectoriser cet agent jusqu'à des cellules cancéreuses et empêcher l'expression de résistance desdites cellules vis-à-vis dudit agent.

**2.** Utilisation selon la revendication 1, **caractérisée en ce que** dans le peptide de formule (I), les acides aminés hydrophobes sont l'alanine, la valine, la leucine, l'isoleucine, la proline, la phénylalanine, le tryptophane, la tyrosine et la méthionine, et les autres acides aminés sont des acides aminés :

- non-hydrophobes qui peuvent être des acides aminés non polaires comme la glycine, ou polaires comme la sérine, la thréonine, la cystéine, l'asparagine, la glutamine, ou
- acides (acide aspartique ou glutamique), ou
- basiques (lysine, arginine ou histidine), ou
- une association d'acides aminés de ces trois catégories.

**3.** Utilisation selon l'une des revendications 1 ou 2, **caractérisée en ce que** le peptide de formule (I) comprend 6 acides aminés hydrophobes et 10 acides aminés non-hydrophobes.

**4.** Utilisation selon la revendication 1, **caractérisée en ce que** dans les peptides de formule (II) ou (III) :

- B est choisi parmi l'arginine, la lysine, l'acide diaminoacétique, l'acide diaminobutyrique, l'acide diaminopropionique, l'ornithine, et
- X est choisi parmi la glycine, l'alanine, la valine, la norleucine, l'isoleucine, la leucine, la cystéine, la cystéine[Acm], la penicillamine, la méthionine, le serine, la thréonine, l'asparagine, la glutamine, la phénylalanine, l'histidine, le tryptophane, la tyrosine, la proline, l'Abu, l'acide amino-1-cyclohexane carboxylique, l'Aib, la 2-aminotétraline carboxylique, la 4-bromophényalanine, tert-Leucine, la 4-colorophénylalanine, la bêta-cyclohexyalanine, la 3,4-dichlorophényalanine, la 4-fluorophényalanine, l'homoleucine, la bêta-homoleucine, l'homophényalanine, la 4-méthylphényalanine, la 1-naphtyalanine, la 2- naphtyalanine, la 4-nitrophényalanine, la 3-nitrotyrosine, la norvaline, la phénylglycine, la 3-pyridyalanine, la [2-thiényl]alanine.

**5.** Utilisation d'un composé répondant à la formule (IV) suivante :

$$A \, (\text{-})_m \, (B)_n \qquad (IV)$$

dans laquelle :

- A représente un peptide tel que défini dans l'une quelconque des revendications 1 à 4,
- B représente un agent anti-cancéreux,
- n est 1 ou plus, et de préférence jusqu'à 10 avantageusement jusqu'à 5,
- $(\text{-})_m$ représente la liaison, entre A et B, où m est 1 ou plus, et de préférence jusqu'à 10 avantageusement jusqu'à 5,

pour la préparation d'un médicament destiné au traitement et/ou la prévention de cancers chimiorésistants, ledit peptide étant associé dans le médicament avec au moins un agent anticancéreux pour vectoriser cet agent jusqu'à des cellules cancéreuses et empêcher l'expression de résistance desdites cellules vis-à-vis dudit agent.

6.  Utilisation selon la revendication 5, **caractérisée en ce que** dans la formule (IV) la liaison (-)$_m$ entre A et B est une liaison covalente, hydrophobe ou ionique, clivable ou non-clivable dans les milieux physiologiques on à l'intérieur de la cellules, ou mélange de celles-ci.

**Patentansprüche**

1.  Einsatz eines Peptids, das einer der folgenden Formeln (I), (II) oder (III) entspricht:

$$X_1\text{-}X_2\text{-}X_3\text{-}X_4\text{-}X_5\text{-}X_6\text{-}X_7\text{-}X_8\text{-}X_9\text{-}X_{10}\text{-}X_{11}\text{-}X_{12}\text{-}X_{13}\text{-}X_{14}\text{-}X_{15}\text{-}X_{16} \quad (I),$$

wobei es sich bei den Rückständen $X_1$ bis $X_{16}$ in der Formel (I) um Aminosäurerückstände handelt, von denen 6 bis 10 wiederum wasserabweisende Aminosäuren sind und $X_5$ ein Tryptophan ist,

$$\text{BXXBXXXXBBBXXXXXXB} \quad (II)$$

$$\text{BXXXBXXXBXXXXBBXB} \quad (III),$$

bei denen in den Formeln (II) und (III):

-   die Gruppen B, die identisch oder unterschiedlich sind, einen Aminosäurerückstand darstellen, bei dem die Seitenkette eine Basengruppe umfasst, und
-   die Gruppen X, die identisch oder unterschiedlich sind, einen aliphatischen oder aromatischen Aminosäurerückstand darstellen,

oder die genannten Peptide der Formeln (I), (II), (III) in rückwirkender Form aus Aminosäuren mit der Konfiguration D und/oder L bestehen oder ein Teil letzterer aus einer Folge von mindestens 5 besteht und vorzugsweise aus mindestens 7 aufeinanderfolgenden Aminosäuren der Peptidformeln (I), (II) oder (III), zur Vorbereitung eines Medikamentes, das für die Behandlung und/oder Vorbeugung von chemioresistentem Krebs bestimmt ist. Das besagte Peptid wird in dem Medikament mit mindestens einem karzinostatischen Stoff verbunden, um diesen Stoff bis zu den Krebszellen zu vektorisieren und um den Widerstandsausdruck der besagten Zellen gegenüber diesem Stoff zu verhindern.

2.  Einsatz gemäß dem Patentanspruch 1, der **dadurch gekennzeichnet ist, dass** es sich bei den in dem Peptid der Formel (I) enthaltenen wasserabweisenden Amonisäuren um Alanin, Valin, Leucin, Isoleucin, Prolin, Phenylalalin, Trytophan, Tyrosin und Methionin handelt und bei den anderen Aminosäuren um folgende Aminosäurearten:

-   nicht wasserabweisende Abminosäuren, die nicht polar sein können, wie zum Beispiel Glycin oder polare Aminosäuren wie zum Beispiel Serin, Threonin, Cystein, Asparagin, Glutamin oder
-   Säuren (Asparagin- oder Glutaminsäuren) oder
-   Basen (Lysin, Arginin oder Histidin) oder
-   eine Verbindung von Aminosäuren mit diesen drei Kategorien.

3.  Einsatz gemäß einem der Patentansprüche 1 oder 2, der **dadurch gekennzeichnet ist, dass** das Peptid der Formel (I) 6 wasserabweisende Aminosäuren und 10 nicht wasserabweisenden Aminosäuren umfasst.

4.  Einsatz gemäß dem Patentanspruch 1, der **dadurch gekennzeichnet ist, dass** in den Peptiden der Formel (II) oder (III):

-   B unter Arginin, Lysin, Diaminosäure, Diamino-Butyral-Säure, Diamino-Propionalsäure, Ornithin ausgewählt wird und

- X unter Glycin, Alanin, Valin, Norleucin, Isoleucin, lLeucin, Cystein, Cystein$^{Acm}$, Penicillamin, Methionin, Serin, Threonin, Asparagin, Glutamin, Phenylalanin, Histidin, Tryptophan, Tyrosin, Prolin, Abu, Amino-1-Cyclohexan-Carboxyl-Säure, Aib, 2-Aminotetralin-Carboxyl. 4-Bromophenylalanin, Tert-Leucin, 4-Chlorophenylalanin, Beta-Cyclohexylalanin, 3,4-Dichlorophenylalanin, 4-Fluorophenylalanin, Homoleucin, Beta-Homoleucin, Homophenylalanin, 4-Methylphenylalanin, 1-Naphtyalanin, 2-Naphtyalanin, 4-Nitrophenylalanin, 3-Nitrotyrosine, Norvalin, Phenylglycin, 3-Pyridyalanin, (2-Thienyl-)Alanin ausgewählt wird.

5. Einsatz einer Zusammensetzung, die der folgenden Formel (IV) entspricht:

$$A\ (\text{-})_m\ (B)_n \qquad\qquad (IV)$$

bei der:

- A ein Peptid ist, das einem der Peptide entspricht, die in einem der beliebigen Patentansprüche 1 bis 4 beschrieben werden,
- B ist ein karzinostatischer Stoff,
- n ist 1 oder größer und hat vorzugsweise einen Höchstwert von 10 und im günstigsten Fall einen Wert von 5,
- $(\text{-})_m$ stellt die Verbindung zwischen A und B dar, in der m gleich 1 oder größer ist und vorzugsweise einen Höchstwert von 10 ist und im günstigsten Fall einen Wert von 5,
- für die Vorbereitung eines Medikamentes, das für die Behandlung und/oder die Vorbeugung von chemioresistentem Krebs bestimmt ist, ist das besagte Peptid in dem Medikament mit mindestens einem karzinostatischem Stoff verbunden, um diesen Stoff bis zu den Krebszellen zu vektorisieren und den Widerstandsausdruck dieser Zellen gegenüber diesem Stoff zu verhindern.

6. Einsatz gemäß dem Patentanspruch 5, der **dadurch gekennzeichnet ist, dass** es sich bei der Formel (IV) der Verbindung $(\text{-})_m$ zwischen A und B um eine kovalente, wasserabweisende oder ionische, spaltbare oder nicht spaltbare Verbindung in den physiologischen Umgebungen oder im Inneren einer Zelle handelt oder um eine Mischung dieser Verbindungen.

**Claims**

1. Use of a peptide according to one of the following formulas (I), (II) or (III):

$$X_1\text{-}X_2\text{-}X_3\text{-}X_4\text{-}X_5\text{-}X_6\text{-}X_7\text{-}X_8\text{-}X_9\text{-}X_{10}\text{-}X_{11}\text{-}X_{12}\text{-}X_{13}\text{-}X_{14}\text{-}X_{15}\text{-}X_{16} \qquad\qquad (I)$$

in this formula (I), the residues $X_1$ to $X_{16}$ are amino acid residues, and 6 to 10 of them are hydrophobic amino acids and $X_6$ is tryptophane,

$$BXXBXXXXBBBXXXXXXB \qquad\qquad (II)$$

$$BXXXBXXXBXXXXBBXB \qquad\qquad (III),$$

in these formulas (II) and (III):

- groups B, identical or different, represent an amino acid residue in which the side chain carries a basic group, and
- groups X, identical or different, represent an aliphatic or aromatic amino acid residue,

or the said peptides with formulas (I), (II), (III) in retro form composed of amino acids in the D and/or L configuration, or a fragment of these amino acids composed of a sequence of at least 5 and preferably at least 7 successive amino acids of peptides with formulas (I), (II) or (III),

for preparation of a medicine to be used for the treatment and/or prevention of chemioresistant cancers, the said peptide in the medicine being associated with at least one anti-cancer agent to vectorize this agent as far as the cancer cells and prevent expression of resistance of the said cells with regard to the said agent.

**2.** Use according to claim 1, **characterised in that** the peptide with formula (I), the hydrophobic amino acids are alanine, valine, leucine, isoleucine, proline, phenylalanine, tryptophane, tyrosine, and methionine, and the other amino acids are:

- non-hydrophobic amino acids that may be non-polar such as glycine, or polar such as serine, threonine, cysteine, asparagine ?, glutamine, or
- acids (aspartic or glutamic acid), or
- basic (lysine, arginine or histidine), or
- an association of amino acids in these three categories.

**3.** Use according to one of claims 1 or 2, **characterised in that** the peptide with formula (I) comprises 6 hydrophobic amino acids and 10 non-hydrophobic amino acids.

**4.** Use according to claim 1, **characterised in that** among the peptides with formula (II) or (III):

- B is chosen among arginine, lysine, diaminoacetic acid, diaminobutyric acid, diaminopropionic acid, ornithine, and
- X is chosen among glycine, alanine, valine, norleucine, isoleucine, leucine, cysteine, cysteine$^{Acm}$, penicillamine, methionine, serine, threonine, asparagine, glutamine, phenylalanine, histidine, tryptophane, tyrosine, proline, Abu, amino-1-cyclohexane carboxylic acid, Aib, carboxyilic 2-aminotetraline , 4-bromophenyalanine, tert-Leucine, 4-chlorophenylalanine, beta-cyclohexyalanine, 3, 4-dichlorophenyalanine, 4-fluorophenyalanine, homoleucine, beta-homoleucine, homophenyalanine, 4-methylphenyalanine, 1-naphthyalanine, 2-naphthyalanine, 4-nitrophenyalanine, 3-nitrotyrosine, norvaline, phenylglycine, 3-pyridyalanine, [2-thienyl] alanine.

**5.** Use of a compound according to formula (IV) below:

$$A (-)_m (B)_n \qquad\qquad (IV)$$

in which:

- A represents a peptide as defined in any one of claims 1 to 4,
- B represents an anti-cancer agent,
- n is 1 or more and is preferably up to 10 and advantageously up to 5,
- $(-)_m$ represents the bond between A and B, where m is 1 or more and preferably up to 10 and advantageously up to 5,

for the preparation of a medicine to be used for treatment and / or prevention of chemioresistant cancers, the said peptide being associated in the medicine with at least one anti-cancer agent to vectorize this agent as far as the cancer cells and prevent expression of resistance of the said cells with regard to the said agent.

**6.** Use according to claim 5, **characterised in that** the $(-)_m$ bond between A and B in formula (IV) is a covalent, hydrophobic or ionic bond, cleavable or non-cleavable in physiological media or inside the cell, or mix of these bonds.

Figure 1 : Préparation de doxorubicine-Succ-peptides

Figure 2 : Préparation de doxorubicine-SMP-3MP-peptides

Doxorubicin

DIEA (2 eq)
DMF

Doxorubicinyl Maleimido Propionate

3MP-Peptide/DMF

Doxo-SMP-3MP-Peptide

EP 1 135 169 B1

Fig.3

SEQ.ID.5

SEQ.ID.6

doxorubicine

100 90 80 70 60 50 40 30 20 10 0

% de pénétration

Fig.4

Fig.5

K562/ADR

doxo+peptide

SEQ.ID.2

doxorubicine

IC50 (uM)

90 80 70 60 50 40 30 20 10 0